# EUROPEAN PATENT SPECIFICATION

(11) **EP 2 635 586 B1**
(45) Date of publication and mention of the grant of the patent: **08.02.2017**
(21) Application number: 11785301.0
(22) Date of filing: 04.11.2011
(51) Int. Cl.: C07D 489/12, C07D 489/08

(54) **METHOD FOR THE MANUFACTURING OF NALTREXONE**
VERFAHREN ZUR HERSTELLUNG VON NALTREXON
PROCÉDÉ POUR LA FABRICATION DE NALTREXONE

(30) Priority: 25.05.2011 DK 201100396; 25.05.2011 US 201161489701 P; 05.11.2010 US 410405 P; 05.11.2010 DK 201001007
(43) Date of publication of application: 11.09.2013
(73) Proprietor: H. Lundbeck A/S, 2500 Valby (DK)
(72) Inventor: DE FAVERI, Carla, I-31010 Farra di Soligo (IT); HUBER, Florian, Anton, Martin, I-30031 Dolo (IT); STIVANELLO, Mariano, I-36015 Schio (IT)
(86) International application number: PCT/DK2011/000128
(87) International publication number: WO 2012/059103

(56) References cited:
- WO-A1-91/05768
- WO-A2-2010/039209

## Description

### Field of the invention

The present invention relates to an improved process for producing naltrexone [17-(cyclopropylmethyl)-4,5α-epoxy-3,14-dihydroxy-morphinan-6-one] from noroxymorphone [4,5-α-epoxy-3,14-dihydroxy-morphinan-6-one] by alkylation with a cyclopropylmethyl halide.

### Background of the invention

Nalmefene is a known opioid receptor antagonist which can inhibit pharmacological effects of both administered opioid agonists and endogenous agonists derived from the opioid system. The clinical usefulness of nalmefene as antagonist comes from its ability to promptly (and selectively) reverse the effects of these opioid agonists, including the frequently observed depressions in the central nervous system and the respiratory system.

Nalmefene has primarily been developed as the hydrochloride salt for use in the management of alcohol dependency, where it has shown good effect in doses of 10 to 40 mg taken when the patient experiences a craving for alcohol (Karhuvaara et al., Alcohol. Clin. Exp. Res., (2007), Vol. 31 No. 7. pp 1179-1187). Additionally, nalmefene has also been investigated for the treatment of other addictions such as pathological gambling and addiction to shopping. In testing the drug in these developmental programs, nalmefene has been used, for example, in the form of a parenteral solution (Revex^{™}).

Nalmefene is an opiate derivative quite similar in structure to the opiate antagonist naltrexone. Advantages of nalmefene compared to naltrexone include longer half-life, greater oral bioavailability and no observed liver toxicity.

Nalmefene can be produced from naltrexone by the Wittig reaction. Methods for preparation of nalmefene from naltrexone by the Wittig reaction has been described by Hanh et al., (J. Med. Chem., 18, 259-262(1975), Mallinckrodt (US 4,751,307), Meltzner et al., (US patent No. 4,535,157) and by H. Lundbeck (WO 2010/136039). By using the above-mentioned methods, the free base of nalmefene is obtained, which subsequently can be converted into the hydrochloride salt by use of conventional methods.

Naltrexone can be produced from noroxymorphone by various direct and indirect alkylation methods. One method is by direct alkylation of noroxymorphone with cyclopropylmethylbromide. This process has been disclosed in general terms by Rice in WO 91/05768. Sanofi-Avensis (WO 2008/034973) describes a process for obtaining naltrexone in 88.6% yield by reacting noroxymorphone hydrochloride with cyclopropylmethylbromide in dimethylacetamide in the presence of sodium hydrogen carbonate. Cilag (WO 2008/138605) describes N-alkylation of noroxymorphone with cyclopropylmethylbromide in N-methyl-pyrrolidone in the presence of sodium hydrogen carbonate. Mallinckrodt (WO 2010/039209) describes N-alkylation of noroxymorphone with cyclopropylmethylbromide in the presence of a protic solvent. Specific examples in WO 2010/039209 describe the addition of water, isopropanol or ethanol as the protic solvent.

There is a need within the field to improve the method of producing highly pure naltrexone and/or to find alternative processes for producing naltrexone. In particular, there is a need for a method that is readily applicable on industrial scale.

### Summary of the invention

The present invention relates to an improved process for producing naltrexone [17-(cyclopropylmethyl)-4,5α-epoxy-3,14-dihydroxy-morphinan-6-one] from noroxymorphone [4,5-α-epoxy-3,14-dihydroxy-morphinan-6-one] by alkylation of noroxymorphone with a cyclopropylmethyl halide in N-ethyl-2-pyrrolidone as depicted in scheme 1 below. 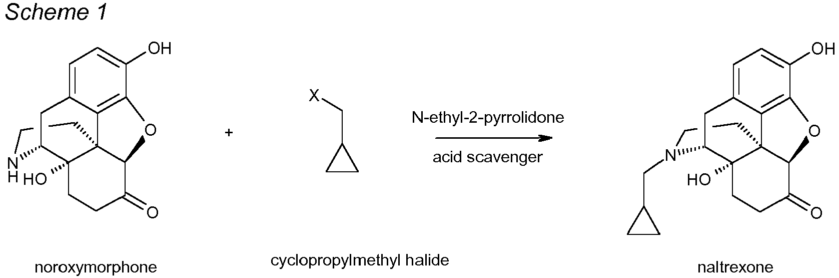 X is chosen from Br, Cl and I

In one embodiment, naltrexone obtained from the process of the invention is further processed e.g. by the Wittig reaction to nalmefene.

In one embodiment, the invention relates to a process for the manufacturing of nalmefene comprising the steps, i) manufacturing of naltrexone by a process of the invention, ii) further processing of naltrexone obtained from i) to nalmefene optionally by the Wittig reaction.

### Definitions

Throughout the description, the terms "naltrexone" and "nalmefene" are intended to include any forms of the compounds, such as the free base and pharmaceutically acceptable salts. The free base and pharmaceutically acceptable salts include anhydrous forms and solvated forms such as hydrates. The anhydrous forms and the solvates include amorphous and crystalline forms. In a particular embodiment naltrexone is in the form of the free base. In a particular embodiment nalmefene is in the form of the hydrochloride.

In the present context, examples of "cyclopropylmethyl halides" include cyclopropylmethyl bromide, cyclopropylmethyl chloride, cyclopropylmethyl iodide. In a particular embodiment, the term "cyclopropylmethyl halide" refers to cyclopropylmethyl bromide.

In the present context, a "non-protic solvent" refers to any non-protic solvent. Non-limiting examples of non-protic solvents include hydrocarbons, ketones, esters and ethers. In a particular embodiment, the term "non-protic solvent" refers to toluene.

In the present context, an "acid scavenger" refers to a compound selected from organic and inorganic bases, and combinations hereof. Examples include borate salts, phosphate salts, bicarbonate salts (such as KHCO₃, NaHCO₃, LiHCO₃ and the like), carbonate salts (such as K₂CO₃, Na₂CO₃, Li₂CO₃ and the like), organic bases (such as pyridine, triethylamine, tripropylamine, tributylamine, N,N-diisopropylethylamine, N-methylmorpholine, N,N-dimethylaminopyridine), and mixtures of any of the above. In a particular embodiment, the term "acid scavenger" refers to KHCO₃. In another particular embodiment, the term "acid scavenger" refers to N,N-diisopropylethylamine.

In the present context, the term "chemically pure" has its normal meaning within the art. Accordingly, an obtained compound which is at least 98% chemically pure comprises at most 2% chemical impurities. The chemical purity may be determined e.g. by HPLC. In the present context chemical purity is determined by % HPLC area.

### Detailed description of the invention

The inventors have found an improved process for producing naltrexone [17-(cyclopropylmethyl)-4,5α-epoxy-3,14-dihydroxy-morphinan-6-one] from noroxymorphone [4,5-α-epoxy-3,14-dihydroxy-morphinan-6-one] by alkylation with a cyclopropylmethyl halide in N-ethyl-2-pyrrolidone. The inventors have found that when running the alkylation in N-ethyl-2-pyrrolidone the reaction kinetics can be controlled efficiently and naltrexone is obtained as a chemically pure compound in a high yield.

In brief, noroxymorphone is mixed with cyclopropylmethyl halide in N-ethyl-2-pyrrolidone. In a preferred embodiment, the reaction is conducted in presence of an acid scavenger. The mixture is heated to a temperature in the range of 30 to 100°C, preferably in the range of 50-70°C, such as in the range of 50-60°C. Reaction time is adjusted in order to have a reasonably high conversion. Optionally, further cyclopropyl methyl halide is added to the mixture and optionally, the mixture is further heated to increase the conversion.

The formed naltrexone is isolated by a method comprising the following steps
a) mixing the reaction mixture with an acid
b) concentrating the reaction mixture
c) mixing the resulting mixture with water
d) optionally mixing the reaction mixture with an acid
e) optionally treating the mixture with charcoal
f) mixing the resulting mixture with a base
g) isolating the resulting solid.
h) optionally suspending the solid in water, mixing with acid followed by mixing with base and then isolating the resulting solid.
i) drying the solid.

In one embodiment, prior to the reaction with cyclopropylmethyl halide; noroxymorphone is mixed with N-ethyl-2-pyrrolidone and a non-protic solvent whereupon the mixture of noroxymorphone, N-ethyl-2-pyrrolidone and non-protic solvent is concentrated for example by distillation under vacuum.

The process of the present invention consistently gives pure naltrexone. The main impurity coming from alkylation of the hydroxyl group in the phenolic moiety is controlled with the process of the invention. The level of the impurity 3-cyclopropylmethylnaltrexone in the isolated naltrexone is below about 0.5% (by area) as measured by HPLC. The process of the invention also allows efficient removal of potentially unreacted noroxymorphone in isolated naltrexone.

Naltrexone prepared according to the method described in this invention can thus be directly used in the preparation of nalmefene e.g. by Wittig reaction.

The use of the terms "a" and "an" and "the" and similar referents in the context of describing the invention are to be construed to cover both the singular and the plural, unless otherwise indicated herein or clearly contradicted by context. For example, the phrase "the compound" is to be understood as referring to various "compounds" of the invention or particular described aspect, unless otherwise indicated.

The description herein of any aspect or aspect of the invention using terms such as "comprising", "having," "including" or "containing" with reference to an element or elements is intended to provide support for a similar aspect or aspect of the invention that "consists of", "consists essentially of" or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

It should be understood that the various aspects, embodiments, implementations and features of the invention mentioned herein may be claimed separately, or in any combination.

### Embodiments according to the invention

In the following, embodiments of the invention are disclosed. The first embodiment is denoted E1, the second embodiment is denoted E2 and so forth.
E1. A process for the manufacturing of naltrexone, comprising reacting noroxymorphone with cyclopropylmethyl halide in the presence of N-ethyl-2-pyrrolidone.
E2. The process according to embodiment 1, wherein the reaction takes place in the presence of an acid scavenger.
E3. The process according to embodiment 2, wherein the acid scavenger is an inorganic or organic base or a mixture thereof.
E4. The process according to any of embodiments 2-3, wherein the acid scavenger is N,N-diisopropylethylamine.
E5. The process according to any of embodiments 2-3, wherein the acid scavenger is potassium bicarbonate.
E6. The process according to any of embodiments 1-5, wherein the cyclopropylmethyl halide is cyclopropylmethyl bromide.
E7. The process according to any of embodiments 1-6, wherein the reaction takes place in the presence of a non-protic solvent.
E8. The process according to any of embodiments 1-7, wherein; prior to the reaction with cyclopropylmethyl halide; noroxymorphone is mixed with N-ethyl-2-pyrrolidone and a non-protic solvent whereupon the mixture of noroxymorphone, N-ethyl-2-pyrrolidone and non-protic solvent is concentrated.
E9. The process according to embodiment 8, wherein said mixture of noroxymorphone, N-ethyl-2-pyrrolidone and non-protic solvent is concentrated by distillation under vacuum.
E10. The process according to any of embodiments 7-9, wherein the non-protic solvent is toluene.
E11. The process according to any of embodiments 1-10, wherein N-ethyl-2-pyrrolidone is used in a weight by weight ratio of 0.5:1 to 10:1 in respect to noroxymorphone.
E12. The process according to embodiment 11, wherein N-ethyl-2-pyrrolidone is used in a weight by weight ratio of 1:1 to 5:1 with respect to noroxymorphone.
E13. The process according to embodiment 12, wherein N-ethyl-2-pyrrolidone is used in a weight by weight ratio of about 3:1 with respect to noroxymorphone.
E14. The process according to any of embodiments 1-13, wherein the molar relationship between noroxymorphone and acid scavenger is from about 1:0.5 to about 1:2.
E15. The process according to embodiment 14, wherein the molar relationship between noroxymorphone and acid scavenger is from about 1:1 to about 1:2.
E16. The process according to embodiment 15, wherein the molar relationship between noroxymorphone and acid scavenger is from about 1:1 to about 1:1.5.
E17. The process according to any of embodiments 1-16, wherein the molar relationship between noroxymorphone and cyclopropylmethyl halide is from about 1:1 to about 1:2.
E18. The process according to embodiment 17, wherein the molar relationship between noroxymorphone and cyclopropylmethyl halide is from about 1:1 to about 1:1.5.
E19. The process according to any of embodiments 1-18, wherein the reaction temperature is in the range of about 30-100°C.
E20. The process according to embodiment 19, wherein the reaction temperature is in the range of about 50-70°C, such as in the range of 50-55°C or 55-60°C or 60-65°C or 65-70°C.
E21. The process according to any of embodiments 19-20, wherein the reaction temperature is in the range of about 50-60°C.
E22. The process according to any of embodiments 1-21, wherein the reaction is running for at least 8 hours; such as in the range of 8-48 hours, such as 8-12 hours, 12-16 hours, 16-20 hours, 20-24 hours, 24-28 hours, 28-32 hours, 32-36 hours, 36-40 hours, 40-44 hours or 44-48 hours.
E23. The process according to embodiment 22, wherein the reaction is running for a range of about 12-24 hours.
E24. The process according to embodiment 23, wherein the reaction is running for a range of about 16-20 hours.
E25. The process according to any of embodiments 1-24, wherein the formed naltrexone is isolated by a method comprising the following steps
   a) mixing the reaction mixture with an acid
   b) concentrating the reaction mixture
   c) mixing the resulting mixture with water
   d) optionally mixing the reaction mixture with an acid
   e) optionally treating the mixture with charcoal
   f) mixing the resulting mixture with a base
   g) isolating the resulting solid.
   h) optionally suspending the solid in water, mixing with acid followed by mixing with base and then isolating the resulting solid.
   i) drying the solid.
E26. The process according to embodiment 25 wherein the acid in steps a), d) and h) is hydrochloric acid.
E27. The process according to any of embodiments 25-26, wherein the base in steps f) and h) is ammonium hydroxide.
E28. The process according to any of embodiments 1-27, wherein the formation of 3-cyclopropylmethyl-naltrexone is less than about 0.5% (by area).
E29. The process according to any of embodiments 1-28, wherein noroxymorphone is used as starting material in form of its free base or its hydrochloride salt.
E30. The process according to any of embodiments 1-29, wherein naltrexone is obtained as the free base.
E31. The process according to embodiment 30, wherein naltrexone free base is obtained as a hydrate.
E32. The process according to embodiment 31, wherein the naltrexone free base hydrate is a monohydrate.
E33. The process according to embodiment 32, wherein the naltrexone free base monohydrate is obtained in crystalline form.
E34. The process according to any of embodiments 1-33, wherein the naltrexone obtained from the process is further processed to give nalmefene.
E35. The process according to embodiment 34, wherein naltrexone obtained from the process is further processed by the Wittig reaction to give nalmefene.
E36. A process for the manufacturing of nalmefene comprising the steps
   i) manufacturing of naltrexone by a process according to any of embodiments 1-33
   ii) further processing of naltrexone obtained from i) to nalmefene optionally by the Wittig reaction.
E37. The process according to embodiment 36 comprising the following subsequent steps
   iii) precipitating nalmefene as a pharmaceutically acceptable salt
   iv) optionally purifying the obtained nalmefene salt.

### Examples

The invention will be illustrated by the following non-limiting examples.

### HPLC Chromatographic conditions

Column:.................................... Zorbax Eclipse XDB, 150 × 4.6 mm, 5 µm or equivalent
Mobile Phase A: ....................... Buffer
Mobile Phase B: ....................... Acetonitrile
Buffer:....................................... 1.1 g of Sodium Octanesulfonate dissolved in 1 L of water, pH adjusted to 2.3 with H₃PO₄.
Column Temperature:...............35°C
Detector:................................... UV at 230 nm
Flow:......................................... 1.2 ml/min
Injection volume:.......................20 µl
Time of Analysis: ...................... 45 minutes

**Table 2: HPLC gradient**

| **Time** | **Mobile Phase A** | **Mobile Phase B** |
|---|---|---|
| 0 | 90 | 10 |
| 45 | 55 | 45 |

### Example 1:

A mixture of noroxymorphone (52.7 g), N-ethyl-2-pyrrolidone (100 ml) and toluene (100 ml) was concentrated under vacuum at 80°C. The mixture was diluted with toluene (100 ml) and concentrated again. The suspension was diluted with N-ethyl-2-pyrrolidone (50 ml). Potassium bicarbonate (24.4 g) and cyclopropylmethyl bromide (29.3 g) were added and the mixture was heated up to 55°C for 23 hours. The composition of the reaction mixture was checked by HPLC (% by area): naltrexone 97.3%, noroxymorphone 1.4%, 3-cyclopropylmethylnaltrexone 0.4%.

### Example 2:

Noroxymorphone (51.5 g) in N-ethyl-2-pyrrolidone (168 ml) and toluene (100 ml) was concentrated under vacuum at 80-85°C. Toluene was added (200 ml) and vacuum distillation repeated. Potassium bicarbonate (24.4 g) and cyclopropylmethyl bromide (29.3 g) were added and the mixture was heated up to 60°C and maintained at that temperature for 22 hours. Further cyclopropylmethyl bromide (2.3 g) was charged and stirred at 60°C for five additional hours. The composition of the reaction mixture was checked by HPLC: noroxymorphone 1.5%, naltrexone 97.4% and 3-cyclopropylmethyl naltrexone 0.3%. The reaction mixture was treated with HCl 10% (88.9 g) and concentrated under vacuum. The mixture was cooled and diluted with water (1580 g). Ammonium hydroxide 4% in water was added over 3 hours obtaining a suspension (pH 9.3). The suspension was stirred and then filtered. The solid was washed with water and dried under vacuum at 60°C obtaining 55.9 g of naltexone. HPLC analysis (% by area): naltrexone 99.0%, noroxymorphone 0.1 %, 3-cyclopropylmethyl naltrexone 0.3%.

### Example 3:

A mixture of noroxymorphone (52.7 g), potassium bicarbonate (24.4 g) and cyclopropylmethyl bromide (30.5 g) in N-ethyl-2-pyrrolidone (150 ml) was heated up 60°C for 17 hours. The composition of the reaction mixture was checked by HPLC (% by area): naltrexone 95.7%, noroxymorphone 2.9%, 3-cycloproylmethylnaltrexone 0.3%.

### Example 4:

Noroxymorphone (52.7 g) in N-ethyl-2-pyrrolidone (100 ml) and toluene (100 ml) was concentrated under vacuum. Toluene was added (100 ml) and vacuum distillation repeated two more times. The mixture was diluted with N-ethyl-2-pyrrolidone. Cyclopropylmethyl bromide (30.5 g) and N,N-diisopropylethylamine (29.2 g) were added and the mixture was heated up to 60°C and maintained at that temperature for 17 hours. The composition of the reaction mixture was checked by HPLC (% by area): naltrexone 95.0%, noroxymorphone 3.3%, 3-cyclopropylmethyl naltrexone 0.3%.

### Example 5:

A mixture of noroxymorphone (60 Kg, 0.209 Kmol), N-ethyl-2-pyrrolidone (180 kg), cyclopropymethyl bromide (36.6 kg) and N,N-diisopropylethylamine (35.1 kg) was heated to 52-57°C for 20 hours and 10 minutes. The mixture was then diluted with a solution prepared by mixing hydrochloric acid 37% (29 kg) and water (79 kg). Low boiling compounds were removed by distillation under vacuum keeping the temperature below 70°C. After cooling to 25-30°C the mixture was further diluted with water (1910 kg). Ammonium hydroxide 4% (199 kg) was then added over three hours till pH=9-10 to precipitate the product. The solid was filtered, washed with water (2×120 kg) and dried under vacuum at 60°C obtaining 68.5 kg of naltrexone (molar yield 93.2%). HPLC analysis (% by area): naltrexone 99.1%, noroxymorphone 0.11%, 3-cyclopropylmethylnaltrexone 0.41%

### Example 6:

A mixture of noroxymorphone (60 Kg, 0.209 Kmol), N-ethyl-2-pyrrolidone (180 kg), cyclopropymethyl bromide (36.6 kg) and N,N-diisopropylethylamine (35.1 kg) was heated to 52-57°C for 18 hours and 30 min. The mixture was then diluted with a solution prepared by mixing hydrochloric acid 37% (29 kg) and water (79 kg). Low boiling compounds were removed by distillation under vacuum keeping the temperature below 70°C. After cooling to 25-30°C the mixture was further diluted with water (1910 kg). Ammonium hydroxide 4% (199 kg) was then added over three hours till pH=9-10 to precipitate the product. The solid was filtered, washed with water (2x120 kg) and dried under vacuum at 60°C obtaining 69 kg of naltrexone (molar yield 89.7%). HPLC analysis (% by area): naltrexone 99.1%, noroxymorphone 0.09%, 3-cyclopropylmethylnaltrexone 0.41 %

### Example 7:

A mixture of noroxymorphone (62 Kg), N-ethyl-2-pyrrolidone (186 kg), cyclopropymethyl bromide (37.8 kg) and N,N-diisopropylethylamine (36.2 kg) was heated to 52-57°C for 24 hours and 45 min. The mixture was then diluted with a solution prepared by mixing hydrochloric acid 37% (30 kg) and water (82 kg). Low boiling compounds were removed by distillation under vacuum keeping the temperature below 70°C. After cooling to 25-30°C the mixture was further diluted with water (1975 kg). Ammonium hydroxide 4% (206 kg) was then added over three hours till pH=9-10 to precipitate the product. The solid was filtered, washed with water (2x124 kg) and dried under vacuum at 60°C obtaining 71.3 kg of naltrexone (molar yield 89.6%). HPLC analysis (% by area): naltrexone 99.45%, noroxymorphone 0.16%, 3-cyclopropylmethylnaltrexone 0.28%

## Claims

1. A process for the manufacturing of naltrexone, comprising reacting noroxymorphone with cyclopropylmethyl halide in the presence of N-ethyl-2-pyrrolidone.

2. The process according to claim 1, wherein the reaction takes place in the presence of an acid scavenger.

3. The process according to claim 2, wherein the acid scavenger is an inorganic or organic base or a mixture thereof.

4. The process according to any of claims 2-3, wherein the acid scavenger is N,N-diisopropylethylamine.

5. The process according to any of claims 1-4, wherein the cyclopropylmethyl halide is cyclopropylmethyl bromide.

6. The process according to any of claims 1-5, wherein; prior to the reaction with cyclo-propylmethyl halide; noroxymorphone is mixed with N-ethyl-2-pyrrolidone and a non-protic solvent whereupon the mixture of noroxymorphone, N-ethyl-2-pyrrolidone and non-protic solvent is concentrated.

7. The process according to any of claims 1-6, wherein N-ethyl-2-pyrrolidone is used in a weight by weight ratio of 0.5:1 to 10:1 with respect to noroxymorphone.

8. The process according to any of claims 2-7, wherein the molar relationship between noroxymorphone and acid scavenger is from about 1:0.5 to about 1:2.

9. The process according to any of claims 1-8, wherein the molar relationship between noroxymorphone and cyclopropylmethyl halide is from about 1:1 to about 1:2.

10. The process according to any of claims 1-9, wherein the reaction temperature is in the range of about 30-100°C.

11. The process according to claim 10, wherein the reaction temperature is in the range of about 50-70°C, such as in the range of 50-55°C or 55-60°C or 60-65°C or 65-70°C.

12. The process according to any of claims 1-11, wherein the formed naltrexone is isolated by a method comprising the following steps
a) mixing the reaction mixture with an acid
b) concentrating the reaction mixture
c) mixing the resulting mixture with water
d) optionally mixing the reaction mixture with an acid
e) optionally treating the mixture with charcoal
f) mixing the resulting mixture with a base
g) isolating the resulting solid.
h) optionally suspending the solid in water, mixing with acid followed by mixing with base and then isolating the resulting solid.
i) drying the solid.

13. A process for the manufacturing of nalmefene comprising the steps
i) manufacturing of naltrexone by a process according to any of claims 1-12
ii) further processing of naltrexone obtained from i) to nalmefene optionally by the Wittig reaction.

14. The process according to claim 13 comprising the following subsequent steps
iii) precipitating nalmefene as a pharmaceutically acceptable salt
iv) optionally purifying the obtained nalmefene salt.

## Patentansprüche

1. Verfahren zur Herstellung von Naltrexon, enthaltend das Reagieren von Noroxymorphon mit Cyclopropylmethylhalid in der Anwesenheit von N-Ethyl-2-pyrrolidon.

2. Verfahren nach Anspruch 1, wobei die Reaktion in Anwesenheit eines Säure-Scavenger stattfindet.

3. Verfahren nach Anspruch 2, wobei der Säure-Scavenger eine anorganische oder organische Base oder eine Mischung daraus ist.

4. Verfahren nach einem der Ansprüche 2-3, wobei der Säure-Scavenger N,N-Diisopropylethylamin ist.

5. Verfahren nach einem der Ansprüche 1-4, wobei das Cyclopropylmethylhalid Cyclopropylmethylbromid ist.

6. Verfahren nach einem der Ansprüche 1-5, wobei vor der Reaktion mit Cyclopropylmethylhalid Noroxymorphon mit N-Ethyl-2-Pyrrolidon und einem nicht-protischen Lösemittel gemischt wird, woraufhin das Gemisch von Noroxymorphon, N-Ethyl-2-Pyrrolidon und dem nicht-protischen Lösemittel konzentriert wird.

7. Verfahren nach einem der Ansprüche 1-6, wobei N-Ethyl-2-Pyrrolidon in einem Gewichtsverhältnis von 0,5:1 bis 10:1 in Bezug auf Noroxymorphon verwendet wird.

8. Verfahren nach einem der Ansprüche 2-7, wobei das molare Verhältnis zwischen Noroxymorphon und Säure-Scavenger von etwa 1:0,5 bis etwa 1:2 beträgt.

9. Verfahren nach einem der Ansprüche 1-8, wobei das molare Verhältnis zwischen Noroxymorphon und Cyclopropylmethylhalid von etwa 1:1 bis etwa 1:2 beträgt.

10. Verfahren nach einem der Ansprüche 1-9, wobei die Reaktionstemperatur im Bereich von etwa 30-100 °C liegt.

11. Verfahren nach Anspruch 10, wobei die Reaktionstemperatur im Bereich von etwa 50-70 °C, wie etwa im Bereich von 50-55 °C oder 55-60°C oder 60-65°C oder 65-70°C liegt.

12. Verfahren nach einem der Ansprüche 1-11, wobei das gebildete Naltrexon durch ein Verfahren isoliert wird, welches die folgenden Schritte umfasst:
a) Mischen des Reaktionsgemischs mit einer Säure
b) Konzentrieren des Reaktionsgemischs
c) Mischen des Reaktionsgemischs mit Wasser
d) optional Mischen des Reaktionsgemischs mit einer Säure
e) optional behandeln des Gemischs mit Holzkohle
f) Mischen des resultierenden Gemischs mit einer Base
g) Isolieren des resultierenden Feststoffs
h) optional Suspendieren des Feststoffs in Wasser, Mischen mit Säure, gefolgt vom Mischen mit Base und anschließend Isolieren des resultierenden Feststoffs
i) Trocknen des Feststoffs.

13. Verfahren zur Herstellung von Nalmefen, enthaltend die Schritte:
i) Herstellen von Naltrexon durch ein Verfahren nach einem der Ansprüche 1-12
ii) weiteres Verarbeiten des aus i) erhaltenen Naltrexons zu Nalmefen, optional durch die Wittig-Reaktion.

14. Verfahren nach Anspruch 13, enthaltend die folgenden nachfolgenden Schritte:
iii) Ausfällen von Nalmefen als pharmazeutisch akzeptables Salz
iv) optional Reinigen des erhaltenen Nalmefen-Salzes.

## Revendications

1. Procédé de fabrication de naltrexone, comprenant la réaction de noroxymorphone avec un halogénure de cyclopropylméthyle en présence de N-éthyl-2-pyrrolidone.

2. Procédé selon la revendication 1, dans lequel la réaction a lieu en présence d'un adsorbant d'acide.

3. Procédé selon la revendication 2, dans lequel l'adsorbant d'acide est une base inorganique ou organique ou un mélange de celle-ci.

4. Procédé selon l'une quelconque des revendications 2 et 3, dans lequel l'adsorbant d'acide est la N, N-diisopropyléthylamine.

5. Procédé selon l'une quelconque des revendications 1 à 4, dans lequel l'halogénure de cyclopropylméthyle est le bromure de cyclopropylméthyle.

6. Procédé selon l'une quelconque des revendications 1 à 5, dans lequel, avant la réaction avec l'halogénure de cyclopropylméthyle, la noroxymorphone est mélangée à de la N-éthyl-2-pyrrolidone et à un solvant aprotique, après quoi le mélange de noroxymorphone, de N-éthyl-2-pyrrolidone et de solvant aprotique est concentré.

7. Procédé selon l'une quelconque des revendications 1 à 6, dans lequel de la N-éthyl-2-pyrrolidone est utilisée dans un rapport pondéral de 0,5:1 à 10:1 par rapport à la noroxymorphone.

8. Procédé selon l'une quelconque des revendications 2 à 7, dans lequel la relation molaire entre la noroxymorphone et l'adsorbant d'acide est d'environ 1:0,5 à environ 1:2.

9. Procédé selon l'une quelconque des revendications 1 à 8, dans lequel la relation molaire entre la noroxymorphone et l'halogénure de cyclopropylméthyle est d'environ 1:1 à environ 1:2.

10. Procédé selon l'une quelconque des revendications 1 à 9, dans lequel la température réactionnelle se situe dans la plage de 30 à 100°C.

11. Procédé selon la revendication 10, dans lequel la température réactionnelle se situe dans la plage d'environ 50 à 70°C, notamment dans la plage de 50 à 55°C ou de 55 à 60°C ou de 60 à 65°C ou de 65 à 70°C.

12. Procédé selon l'une quelconque des revendications 1 à 11, dans lequel la naltrexone formée est isolée par un procédé comprenant les étapes consistant à :
a) mélanger le mélange réactionnel à un acide,
b) concentrer le mélange réactionnel,
c) mélanger le mélange obtenu à de l'eau,
d) mélanger éventuellement le mélange réactionnel à un acide,
e) traiter éventuellement le mélange avec du charbon de bois,
f) mélanger le mélange obtenu à une base,
g) isoler le solide obtenu,
h) mettre éventuellement en suspension le solde dans de l'eau, mélanger à un acide, le tout étant suivi d'un mélange à une base, puis de l'isolement du solide obtenu, et
i) sécher le solide.

13. Procédé de fabrication de nalméfène comprenant les étapes consistant à :
i) fabriquer la naltrexone par un procédé selon l'une quelconque des revendications 1 à 12,
ii) traiter en outre la naltrexone obtenue sous i) en nalméfène éventuellement par la réaction de Wittig.

14. Procédé selon la revendication 13, comprenant les autres étapes consistant à :
iii) précipiter le nalméfène sous la forme d'un sel acceptable au plan pharmaceutique, et
iv) purifier éventuellement le sel de nalméfène obtenu.
